(19) Europäisches Patentamt
European Patent Office
Office européen des brevets

(11) **EP 3 193 838 B1**

(12) **EUROPEAN PATENT SPECIFICATION**

(45) Date of publication and mention of the grant of the patent:
**24.10.2018 Bulletin 2018/43**

(21) Application number: **15763626.7**

(22) Date of filing: **18.09.2015**

(51) Int Cl.:
*A61K 9/16* (2006.01)     *A61K 38/18* (2006.01)
*A61K 38/19* (2006.01)     *A61K 9/20* (2006.01)
*A61K 9/51* (2006.01)     *A61K 9/19* (2006.01)

(86) International application number:
**PCT/EP2015/071410**

(87) International publication number:
**WO 2016/042121 (24.03.2016 Gazette 2016/12)**

(54) **SPRAY-FREEZE DRYING OF POLYELECTROLYTE NANOPARTICLES CONTAINING THE PROTEIN DRUG**

SPRÜHGEFRIERTROCKNUNG VON POLYELEKTROLYTNANOPARTIKELN MIT PROTEINARZNEIMITTEL

LYOPHILISATION PAR VAPORISATION DE NANOPARTICULES DE POLYÉLECTROLYTE CONTENANT LE MÉDICAMENT DE LA PROTÉINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.09.2014 EP 14185421**

(43) Date of publication of application:
**26.07.2017 Bulletin 2017/30**

(73) Proprietor: **LEK Pharmaceuticals d.d.
1526 Ljubljana (SI)**

(72) Inventors:
• **CEGNAR, Mateja**
  **1526 Ljubljana (SI)**
• **AVANZO, Matej**
  **1526 Ljubljana (SI)**
• **KERC, Janez**
  **1526 Ljubljana (SI)**
• **MIKLAVZIN, Ana**
  **1235 Radomlje (SI)**

(74) Representative: **Greiner, Elisabeth
df-mp Dörries Frank-Molnia & Pohlman
Patentanwälte Rechtsanwälte PartG mbB
Theatinerstraße 16
80333 München (DE)**

(56) References cited:
**US-A1- 2009 011 008     US-B1- 6 284 282**

• **HICKEY A J ET AL: "Dry powder nasal vaccines as an alternative to needle-based delivery", CRITICAL REVIEWS IN THERAPEUTIC DRUG CARRIER SYSTEMS, BEGELL HOUSE PUBLISHING INC, US, vol. 26, no. 1, 1 January 2009 (2009-01-01), pages 1-27, XP009135117, ISSN: 0743-4863**

**Description**

**Background**

In the last few years, colloidal drug delivery systems and especially nanoparticles (NPs) have received increasing attention. Despite several advantages of NPs, their chemical and/or physical instability in dispersion remains their main issue. To avoid this problem, water or other solvent(s) has to be removed from the dispersed system. Thus, the drying of nanoparticle dispersions represents an important operation in the production of nanoparticles. Only the dried product ensures long-term stability and also enables the further formulation into solid dosage forms.

The most commonly used methods to remove the water or solvents from the dispersed system are freeze-drying (lyophilisation) and spray-drying. Until now, mainly the classical freeze-drying procedure was studied for drying nanoparticulate dispersions, employing different excipients that either provided support for the NPs, e.g., bulking agents, or replaced the water bonding with amorphous NPs (stabilizers). By selecting specific excipients in adequate amounts, the process of freeze-drying proved to be suitable for drying NPs as well as biopharmaceuticals, because their initial properties in terms of NP-size and protein biological activity were retained after reconstitution in water.

In one study, poly(caprolactone) (PCL) nanoparticles were produced using a w/o/w double emulsification method in order to study the effect of freeze-drying adjuvants on the production of thermally sensitive polymeric nanoparticle aggregates. Mannitol and poly(vinyl alcohol) (PVA) were used as an adjuvants. PCL nanoparticles were physically dispersed in a porous mannitol matrix when mannitol was used as adjuvant, whereas the use of PVA as adjuvant led to coating the nanoparticles surface. The aqueous re-dispersibility of PCL nano-aggregates was enhanced with both adjuvants. Good flowability and effective aerosolization from the inhaler was also shown for both adjuvants.

Apart from classical freeze-drying, spray-freeze drying (SFD) is a rather new technique for removing water from dispersed systems. However, the SFD technique has not yet been investigated for drying NP dispersions and is also not used commercially yet. Also, the use of the newly developed SFD method for drying systems containing protein drugs could be difficult, as the sheer forces that occur during that process might lead to the denaturation of the proteins. US6284282B describes a method of SFD for producing a dry powder of a therapeutic protein for pulmonary administration, which uses the protein particles together with minimum amounts of protectants (cryo- and lyoprotectants) such as carbohydrates (mannitol, trehalose, sucrose), aminoacids (glycine, histide, L-arginine), etc. The authors compared the SFD particles with spray-dried particles. Large, porous particles with aerodynamic particle sizes between 6 $\mu$m and 8 $\mu$m and significantly improved aerosol performance were produced with the SFD method compared to spray dried powders.

A further development of the SFD method is the so called spray-freezing into liquid (SFL), where a feed solution containing an active pharmaceutical ingredient and pharmaceutical excipient(s) is atomized beneath the surface of a cryogenic liquid (e.g., liquid nitrogen). It was observed that the dissolution rates of SFL micronized powders of the two poorly soluble drugs carbamazepine and danazol were enhanced when powders were prepared with the SFL method. This method was also used to produce stable peptide particles of insulin with 3 $\mu$m in diameter.

The influence of the atomization on protein aggregation and enzyme activity of reconstituted lysozyme particles produced by the two cryogenic technologies SFL and SFD using the same excipients (trehalose, Tween 20) was studied. Both, SFL and SFD resulted in highly porous microparticle aggregates of lysozyme nanoparticles (observed by SEM). The smaller degree of protein aggregation and smaller losses in enzyme activity for the SFL process compared to the SFD process were primarily due to the spraying step. SFL powders compared to SFD powders were more stable.

The effect of the atomization conditions on the size and stability of the resulting SFD protein particles has also been studied. In this regard, the atomization variables were explored for excipient-free (no zinc added) and zinc-complexated bovine serum albumin (BSA). The release of SFD BSA was assessed *in vitro* from the BSA loaded poly(lactide-co-glicolide) (PLG) microspheres prepared by a non-aqueous cryogenic process. The most significant atomization parameter affecting the particle size seemed to be the mass flow ratio (mass of atomization $N_2$ relative to that for liquid feed). The particle size was found to be inversely related to the specific surface area and the amount of formed protein aggregates. Zinc complexation reduced the specific surface area and stabilized the protein against aggregation. The reduction of the protein particle size was beneficial in reducing the initial release (burst) of the BSA encapsulated in PLG microspheres.

In another study, SFD and spray-drying were used and compared during the development of different nanocomposite microcarriers (NCMs) as dry inhalation powder. The spray-dried NCMs (SD-NCMs) with equivalent compositions were used as a control. The particle size of spray-freeze dried NCMs (SFD-NCMs) was smaller (12.3 $\mu$m, 10.6 $\mu$m and 9.3 $\mu$m for ethyl cellulose (EC), poly(meth)acrylate and poly(DL-lactide-co-glicolide) (PLGA), respectively, and 7.2 $\mu$m, 6.2 $\mu$m and 5.2 $\mu$m for EC, poly(meth)acrylate and PLGA SD-NCM, respectively). The SFD-NCMs were spherical in shape and had a 10-times lower bulk density (0.02 g/cm³) compared to the corresponding SD-NCMs; they also showed very good flowability compared to the SD-NCMs (Carr index between 6-14 for SFD-NCMs and 22-28 for SD-NCMs). The specific surface area of the SFD-NCMs was much larger than that of the SD-NCMs, which is also reflected by the

$S_f/S_i$ values after reconstitution in water. All SFD-NCMs showed complete reconstitution in water as reflected by the $S_f/S_i$ ratio ($S_f/S_i \approx 1$), whereas the SD-NCM showed a $S_f/S_i$ ratio of $S_f/S_i$ = 5.2 and 2.9 for the SD-NCM based on PLGA and EC nanoparticles, respectively.

An SFD technology is also reported for the preparation of composite particles containing the poorly water soluble drug tolbutamide (TBM) and water-soluble polymer, hydroxypropylmethylcellulose (HPMC). SFD particles were physico-chemically characterized in terms of solubility improvement. It was reported that the SFD particles had a unique structure with many small pores and a particle size of from 55 $\mu$m for a 0.9% solution of TBM-HPMC to 178 $\mu$m for a 13.5% solution. The specific surface area was increased when the concentration of the solution was increased, and was from 18 $m^2/g$ to 28 $m^2/g$. The release profile of TBM from SFD composite particles was considerably improved and reached 100% release of TBM in the first 30 minutes of the dissolution test.

The morphology of SFD particles of a whole inactivated virus influenza vaccine prepared by using inulin, dextran and a mixture of dextran and trehalose as protectants revealed that all three sugars formed highly porous spherical particles with interconnected pores and particle sizes ranging from 1 $\mu$m to 10 $\mu$m. During a 3 month stability test at temperatures up to 30°C, the particle size distribution and specific surface area remained the same. However, when stored at 40°C, the size and specific surface area of SFD particles of inulin and dextran/trehalose was reduced. Immunization experiments, where 3 months old SFD powders were used, demonstrated that the immunogenicity of whole inactivated virus influenza vaccine was not decreased during storage at 30°C.

SFD powder formulations of the hepatitis B surface antigen (HBsAg) containing inulin or a mixture of dextran and trehalose without aluminium were prepared to decrease the cold-chain dependency (hepatitis B vaccine needs to be stored and transported under refrigerated conditions, i.e., 2-8°C). The stability of HBsAg in the amorphous powder formulations was strongly improved during storage both at room temperature and at 60°C for three months, compared to a liquid plain and an aluminium hydroxide adjuvanted formulation. Although the immune response was improved when the sugar was spray-freeze dried, it was not as high as after the administration of the aluminium adjuvanted HBsAg formulation, but the spray-freeze dried formulation elicited a more balanced Th1/Th2 immune response than the liquid formulation.

In another study, insulin particles for needle-free ballistic powder delivery were prepared by an SFD method. First solutions of insulin or a mixture of insulin and trehalose were spray-freeze dried using a 60 kHz ultrasound nozzle. In the next step, insulin-loaded microspheres with immediate release properties were prepared by spray-freeze drying of insulin nano-suspensions. Trehalose, mannitol and dextran were used as protectants in high concentrations (> 300 mg/ml). Insulin loaded microspheres were spray-freeze dried at 25 kHz and 48 kHz ultrasound nozzle. SFD nanoparticles of insulin and insulin-trehalose were highly porous and were broken up into nanoparticles of 238 nm and 285 nm, respectively, by subsequent homogenization with an Ultra-Turrax tool. SFD microspheres prepared with a concentrated solution of trehalose, mannitol and two dextrans (10 and 150 kDa) allowed for a high insulin loading up to 36.7%.

Up to now, for the drying of NPs, the classical freeze-drying has been the method of choice, whereas the SFD method is a rather new technique, which has not been appropriately investigated and used for drying nanoparticulate dispersions. However, the dry product formed after classical freeze-drying typically exhibits a cake-like structure that needs to be rehydrated for further use.

US2009/0011008 A1 discloses preparations of nanoparticles composed of chitosan/poly-glutamic acid/ insulin.

The drying of nano-dispersions, wherein the NPs are polyelectrolyte complex (PEC) nanoparticles bears an additional challenge, which lies in the nature of the sensitive PECs, and is governed mostly by media parameter limitations such as pH, ionic strength, temperature etc., which are known to vary during the process of freezing and drying. This is even truer for PECs containing protein or peptide drugs, which require very mild processing conditions to prevent their denaturation.

There is hence an ongoing need for methods for drying dispersions of PEC nanoparticles, and in particular, PEC nanoparticles containing protein drugs, which result in a dry, freely flowable powder that is suitable for further formulation into solid dosage forms, while preserving the initial properties of the nanoparticles after reconstitution into a nano-dispersion, i.e., particle size and biological activity of the drugs.

The present invention meets that need by providing a spray-freeze drying (SFD) process that employs minimum amounts of selected excipients to dry the dispersions of the PEC nanoparticles comprising protein drugs, which results in a free flowing powder fit for further conversion into solid formulations while preserving the nanoparticle size as well as the protein biological activity after reconstitution of the dried product in water.

## Summary of the invention

PEC nanoparticles are physically and/or chemically instable in aqueous dispersions. On the one hand, PEC nanoparticles are prone to aggregation or fusion of particles; on the other hand, the polyelectrolyte complexes may dissociate, or the polymers may hydrolyze; the loss of protein drug activity is also a problem. In order to improve the physical and chemical stability of the PEC nanoparticles, water has to be removed. The classical freeze-drying processes yield products that exhibit a cake-like structure that are not easy to handle, and that cannot be formulated further into solid dosage forms. A novel spray-freeze-drying (SFD) process for drying PEC nanoparticles is provided herein, which yields a powdered product that does not cake and has adequate flowability properties suitable for further formulation into solid dosage forms such as tablets or capsules.

The SFD process of the present invention is characterized by the use of a unique combination of sugar excipients in minimum amounts for drying PEC nanoparticles, and in particular protein loaded PEC nanoparticles.

It has been surprisingly found that a combination of one sugar that crystallizes during lyophilisation, and another sugar that remains in the amorphous state after lyophilisation, is most efficient for providing an SFD powder with good flowability properties.

## Figure Legends

Figure 1: SEM images of SFD particles prepared from primary dispersions containing PEC NPs and 4 w/v % mannitol (4.8 w/w % NPs per SFD powder).
Figure 2: SEM images of SFD particles prepared from primary dispersions containing PEC NPs and 4 w/v % dextran (4.8 w/w % NPs per SFD powder). Image at the bottom right corner represents a sample that was exposed to air for one day.
Figure 3: SEM images of SFD particles prepared from primary dispersions containing PEC NPs and 4 w/v % mannitol and dextran (1:1) (4.8 w/w % NPs per SFD powder).

## Detailed description of the invention

The drying of nanoparticle dispersions represents an important operation in the production of nanoparticles. Only the dried products ensure long-term stability and may be formulated further into solid dosage forms. However, until now, the conversion of a dispersion of NPs, in particular PEC nanoparticles or protein drug loaded PEC nanoparticles, into a dry, freely flowing powder, wherein the protein drug activity is retained after redispersion, has represented an unmet need.

The present invention relates to a process for preparing a spray-freeze-dried powder of polyelectrolyte complex (PEC) nanoparticles comprising the steps of

(a) providing a primary dispersion comprising PEC nanoparticles, at least one sugar that crystallizes during lyophilisation, and at least one sugar that remains in the amorphous state, wherein said at least one sugar that crystallizes during lyophilisation is mannitol, and said at least one sugar that remains in the amorphous state is dextran,
(b) spray-freeze drying said primary dispersion, and
(c) isolating the obtained spray-freeze dried powder, wherein said PEC nanoparticles comprise one or more protein drugs.

The invention also relates to a spray-freeze dried powder obtained by said process and to a pharmaceutical composition comprising said spray-freeze dried powder. The invention further relates to said spray-freeze dried powder or composition for use in therapy or diagnosis, as well as to the use of said spray-freeze dried powder or composition for the preparation of a medicament.

Nanoparticles made by polyelectrolyte complexation (PEC) have shown a great potential in formulating biological drugs since this method avoids the application of harsh technological procedures that are usually harmful to sensitive biomolecules such as protein or peptide drugs. PECs are formed in dilute aqueous solutions of oppositely charged polyelectrolytes that have the ability to spontaneously associate into nanocomplexes upon mixing under gentle stirring. However, the formed PEC nanoparticles as well as the protein drugs comprised therein are physically and/or chemically unstable in the resulting aqueous dispersions, so that the water has to be removed. The classical freeze-drying method would be the most suitable method for drying biologically active substances such as protein drugs, as it provides a solid product having a preserved porous inner structure, which may be quickly and easily reconstituted in water, where it forms a homogenous system in solution. However, the lyophilisates obtained by conventional freeze-drying methods exhibit cake-like voluminous, coherent structures and are therefore entirely unsuited for further galenic manipulation into solid dosage forms. Although it might be possible to crumb the cake like product into smaller pieces, a free-flowing powder cannot be obtained with any of the methods of crumbling or grinding. Usually flakes of different sizes are obtained, which are very light, fluffy, electrostatic or hygroscopic, and impossible to manipulate. Their use for further formulation into solid dosage forms is therefore simply not possible.

*Spray-freeze-drying (SFD) process of the invention*

A novel spray-freeze-drying (SFD) process is provided herein, which yields a powdered product with adequate flowability properties suitable for further manipulation or processing, e.g. tableting, or encapsulation of the powder etc. To the best of our knowledge, so far, no studies have been performed on spray-freeze-drying (SFD) of PEC nanoparticles, or PEC nanoparticles containing a protein drug. The drying of this specific type of colloidal system is highly challenging. An inadequate reconstitution of the dried PEC NPs, or PEC nanoparticles comprising a protein drug, in aqueous media may lead to the formation of large particle aggregates, or the PECs may dissociate due to their weak and reversible interactions, which are governed mostly by media parameter such as pH, ionic strength, or temperature etc. If this happens, the beneficial colloidal properties of the nanosystem are lost. Also, the biological activity of the entrapped protein must be preserved during and after the SFD process, and after the reconstitution of the dried SFD PEC NPs in water.

In order to obtain lyophilized PEC nanoparticles, in particular protein drug loaded PEC nanoparticles, that do not have a cake-like structure, and at the same time provide the desired properties of the lyophilisate (preserved inner porous structure, quick and easy reconstitution in water), a modified spray-freeze drying (SFD) technique is provided herein, which uses a particular combination of sugar excipients. It has been surprisingly found that a combination of at least one sugar that crystallizes during lyophilisation and at least one sugar that remains in the amorphous state, as excipients during SFD is most efficient for providing an SFD powder with good flowability properties.

In the SFD process of the present invention, a primary dispersion comprising polyelectrolyte complex (PEC) nanoparticles that may comprise one or more protein drug(s), at least one sugar that crystallizes during lyophilisation, and at least one sugar that remains in the amorphous state, is formed in a first step. In a second step, said primary dispersion is spray-freeze dried, and the spray-freeze dried powder is isolated.

The choice and amount of sugar excipients used for forming the primary dispersion according to the first step of the process is critical. On the one hand, these excipients should be able to preserve the initial properties of the nanosystem in terms of ease of reconstitution in water to give a homogenous system in solution, wherein the particles have a preserved inner porous structure. On the other hand, they should not interfere with the biological activity of the protein drug(s) comprised in the PEC nanoparticles. Several sugars in different concentrations and combinations were tested as excipients for the spray-freeze-drying of PEC nanoparticles, and the resulting powdered lyophilisates were evaluated according to ease of manipulation (behaviour during handling such as electrostaticity, hygroscopicity etc.), flowability properties (angle of repose and Carr's compressibility index) and particle size and integrity of PECs after rehydration of SFD lyophilisates in water.

It has been surprisingly found that the use of a unique combination of at least one sugar that crystallizes during lyophilization, and at least one sugar that remains in the amorphous state, as excipients in the primary dispersion is most efficient for providing a SFD powder with good flowability properties and preserved protein biological activity. Suitable sugars that crystallize during lyophilisation are, for example, mannitol and sorbitol, or combinations thereof. Suitable sugars that remain in the amorphous state are lactose, trehalose, sucrose, dextran, dextrin, maltodextrin or cyclodextrin or combinations thereof, preferably, sugars of longer chains, i.e., polysaccharides such as dextran, dextrin, maltodextrin or cyclodextrin or combinations thereof. While any combination of at least one sugar that crystallizes during lyophilisation and at least one sugar that remains in the amorphous state may be used as excipients for providing the primary dispersion in the process disclosed herein, the process of the present invention uses a combination of mannitol as a sugar that crystallizes during lyophilisation with dextran as a sugar that remains in the amorphous state. The spray-freeze-dried powder based on a primary dispersion comprising PEC nanoparticles and a combination of mannitol and dextran as excipients exhibited the least electrostatic and/or hygroscopic properties among all the excipients tested. The use of either mannitol or dextran alone was not as efficient as their combination. Although they proved to provide good flowability (mannitol) or re-dispersibility (dextran) of the SFD product when tested alone, separately they could not provide all the required characteristics of the dried product. It seems that their combination has a synergistic impact on achieving the desired properties of the SFD product.

In one embodiment, a primary dispersion comprising PEC nanoparticles, mannitol and dextran is provided in the first step of the process of the present invention. In one embodiment, a primary dispersion comprising PEC nanoparticles comprising a protein drug, mannitol and dextran is provided in the first step of the process provided herein.

The amount of the sugar excipients used for preparing the primary dispersion is also important. The excipients should serve as NP-carriers and be present in amounts that are sufficiently high to enable appropriate further manipulation/formulation. At the same time, they should be present in amounts that enable a high loading of NPs per dried product. In this regard, lesser amounts of excipients are preferred to enable a higher loading of NPs per dried product.

The total amount of the at least one sugar that crystallizes during lyophilisation, and the at least one sugar that remains in the amorphous state in the primary solution is, for example, from about 2 w/v % to about 20 w/v %, or from about 3 w/v % to about 15 w/v %, or from about 3 w/v % to about 10 w/v %, or from about 3 w/v % to about 9 w/v%, or from about 3 w/v % to about 8 w/v %, or from about 3 w/v % to about 7 w/v %, or from about 3 w/v % to about 6 w/v % or from about 3 w/v % to about 5 w/v %, or from about 4 w/v % to about 5 w/v %, or 2 w/v %, 3 w/v %, 4 w/v %, 5 w/v %, 6 w/v %, 7 w/v %, 8 w/v %, 9 w/v %, 10 w/v %, 11 w/v %, 12 w/v %, 13 w/v %, 14 w/v %, 15 w/v %, 16 w/v %, 17 w/v %, 18 w/v %, 19 w/v % or 20 w/v %. If a combination of mannitol as a sugar that crystallizes during lyophilisation and dextran as a sugar that remains in the amorphous state is used for preparing the primary dispersion, the most effective total amount of mannitol and dextran in the primary dispersion before SFD is from about 2 w/v % to about 6 w/v %, preferably from about 3 w/v % to about 5 w/v %, and most preferably about 4 w/v %. The use of 4 w/v % of mannitol and dextran in the primary dispersion results in 5.1 w/w % NPs per dried product. The use of too high amounts of excipients in the primary dispersion results in a lower loading of NPs per dried product, which is not warranted. On the other hand, the use of too low amounts of excipients in the primary dispersion results in limited redispersibility after reconstitution of the dry powder in water leading to an increase in nanoparticle size and/or to large particle aggregates after redisperison. Also, the best flowability of the powder is obtained with higher amount of excipients (see also Table 3).

As regards the ratio of the sugar that crystallizes during lyophilisation and the sugar that remains in the amorphous state in the primary dispersion, these sugars may be present in any ratio. For example the ratio of the sugar that crystallizes during lyophilisation and the sugar that remains in the amorphous state in the primary dispersion may be any ratio from 1:2 to 2:1, e.g., 1:2, 1.1:1.9, 1.2:1.8, 1.3:1.7, 1.4:1.6, 1:1, 1.6:1.4, 1.7:1.3, 1.8:1.2, 1.9:1 or 2:1. A ratio of 1:1 is, however, preferred. Hence, if mannitol and dextran are used as excipients in the primary dispersion, they may be present in any ratio between 1:2 to 2:1, e.g., 1:2, 1.1:1.9, 1.2:1.8, 1.3:1.7, 1.4:1.6, 1:1, 1.6:1.4, 1.7:1.3, 1.8:1.2, 1.9:1 or 2:1; however, it is preferred that they are present in the primary dispersion in equal amounts (i.e., in a ratio of 1:1).

The PEC nanoparticles present in the primary dispersion formed in the first step of the process provided herein may comprise one or more protein drugs. Any protein drug may be used for that purpose. Suitable protein drugs may, for example, be selected from G-CSF, EPO, interferon alpha, interferon beta, HGH (human groh hormone), and other cytokines. G-CSF and EPO are, however, preferred protein drugs.

The term "G-CSF" as used herein refers to Granulocyte-Colony Stimulating Factor and may be a natural or recombinant G-CSF, recombinant human G-CSF (rhG-CSF), or any protein or peptide having *in vivo* biological activity of the G-CSF glycoprotein, e.g., a wild type or mutant G-CSF, a G-CSF peptidomimetic, or a G-CSF fragment. The species from which G-CSF is derived can be animal, mammal or human species. Human G-CSF is, however, preferred. Human G-CSF comprises the known human Granulocyte-Colony Stimulating Factor having 174 amino acids. It further comprises human G-CSF in its naturally glycosylated state, i.e., including all carbohydrate side chains, in particular glycosylation at Thr133. The abbreviation "G-CSF" as used herein stands for G-CSF as defined above.

The term "EPO" as used herein refers to erythropoietin and may be a natural or recombinant EPO, recombinant human EPO (rhEPO), or any protein or peptide having *in vivo* biological activity of the EPO glycoprotein, e.g., a wild type or mutant EPO, an EPO peptidomimetic, an EPO fragment, or an EPO conjugate as provided herein. The species from which EPO is derived can be animal, mammal or human species. Human EPO is, however, preferred. Human EPO comprises the known human erythropoietin having 165 amino acids after posttranslational cleavage of the N-terminal signal peptide of 27 amino acids and the C-terminal arginine. It further comprises human EPO in it's naturally glycosylated state, i.e., including all carbohydrate side chains.

The spray-freeze drying of the second step is performed by spraying the primary dispersion formed in the first step through an ultrasonic nozzle into a chamber filled with a cryogenic liquid such as liquid nitrogen. The tiny droplets produced are quickly frozen because of the critically low temperature applied. When the spraying process is completed, the frozen suspension in the cryogenic liquid is transferred into a lyophilizer and dried for an appropriate period of time until a dried particulate powder is obtained.

The process for drying PEC- nanoparticles or protein loaded PEC nanoparticles of the present invention using the unique combination of sugar excipients in appropriate, minimum amounts yields a freely flowable powder. The SFD particles obtained by the process of the present invention have a mean particle size of from about 10 nm to about 500 $\mu$m, preferably from about 100 nm to about 300 $\mu$m, and more preferably from about 1 $\mu$m to about 200 $\mu$m.

The SFD product obtained by the method of the present invention does not exhibit the cake-like structure as obtained by classical freeze-drying processes; it is not electrostatic and hygroscopic and may readily be converted further into pharmaceutical compositions, e.g., into any solid dosage forms such as tablets or capsules, or any other conceivable solid dosage forms. For example, the freeze-dried powder can be compressed into tablets, or encapsulated into capsules.

After reconstitution of the SFD powder produced according to the process provided herein, or of the pharmaceutical compositions made of said SFD powder (e.g., solid dosage forms such as tablets or capsules etc), in water, the particle size of the PEC nanoparticles is preserved and is less than about 1000 nm, preferably less than about 500 nm, and the reconstituted dispersion retains its characteristic turbidity. For example, the tablets compressed from powdered lyophilisates were reconstituted in water and analysed again for particle size and integrity of the PEC nanoparticles. Like the freeze-dried powder, the solid dosage forms, e.g., the tablets, retain nanoparticle size and integrity after reconstitution in water.

Moreover, the process of the present invention results in a product wherein the biological activity of the protein incorporated in PEC nanoparticles is preserved. The proteins released from the PEC nanoparticles after rehydration of the SFD powder prepared according to the process of the present invention, or from the pharmaceutical compositions made of said SFD powder (e.g., from solid dosage forms such as tablets or capsules etc), retain their biological activity.

The SFD powder obtained by the process of the present invention, as well as the pharmaceutical compositions made from the said SFD powder are suitable for use in therapy or diagnosis, and may also be used for the preparation of a medicament.

The present invention is demonstrated in the following by exemplarily using the protein drug G-CSF, which is entrapped between two polyelectrolyte polymers having opposite charges, namely chondroitin sulphate as negatively charged and chitosan as positively charged polymer. Of course, the process of the present invention may also be applied to PEC nanoparticles comprising any other proteins, and to polyelectrolyte protein complexes using polyelectrolyte polymers other than the ones used in the examples below. Suitable polyelectrolyte polymers as well as other protein or peptide drugs are known to the skilled artisan.

**Preparation of polyelectrolyte complexes**

Polyelectrolyte complexes were prepared by a two-step process. Briefly, a protein solution (GCSF, 4 mg/ml, 1.25 ml) was added in a drop-wise manner to the first polymer solution (chondroitin sulphate, 2 mg/ml, 10 ml), which led to complex formation. After stirring for about 20 minutes, the second polymer with an opposite charge to that of the first polymer was added (chitosan, 2 mg/ml, 4.5 ml), which resulted in formation of polyelectrolyte complex (PEC) nanoparticles. The NPs formed were stirred for additional 40 minutes for complete recovery and then analysed.

**Characterisation of polyelectrolyte complexes**

The PECs were characterized with regard to the mean particle diameter, polydispersity, average scattering intensity (particle concentration) and zeta potential by light scattering techniques (dynamic / electrophoretic light scattering : DLS/ELS) using e.g. Zetasizer Nano ZS ZEN 3600 (4mW He-Ne laser, 633nm) from Malvern instruments, UK. For data analysis, a viscosity (0.8863mPa·s) and a refractive index (1.330 at 633nm) of distilled water at 25°C were used. The particle size measurements obtained are based on intensity.

**Spray-freeze-drying (SFD) of polyelectrolyte complexes**

Mannitol, trehalose, sucrose, dextran, maltodextrin, poloxamer 188, polyvinylpyrrolidone K-25 and PEG 6000 were used as SFD excipients. They were evaluated alone, and in binary and ternary mixtures, which were prepared with equal amounts of each excipient. They were added to the primary NP-dispersion before SFD in their final concentration 1 w/v %, 2 w/v %, 3 w/v % and 4 w/v % and analysed again with DLS/ELS.

The primary NP-dispersion with the excipient(s) was fed via a controlled syringe pump at 2 ml/min through an ultrasonic nozzle powered by a Sonotek 25 kHz ultrasonic generator (Sonotek, USA). The atomized droplets fell 10 cm, based on gravity, into liquid nitrogen during stirring on a magnetic stirrer. After the spraying of the dispersion was complete, the excess liquid nitrogen was allowed to evaporate and the frozen droplets were lyophilized in a freeze-dryer Lio-5P LT (Kambic, Slovenia). Drying was performed at 0.003 mbar pressure with a condenser temperature of -100°C for 18 h. Shelf temperature gradually increased to 25°C over 18 h. The SFD products were stored in a desiccator at room temperature and 30% RH before characterisation.

Among all tested excipients, dextran and mannitol, either alone or in combination, provided suitable lyophilisates with most beneficial properties, which showed complete re-dispersibility, which is one of the key parameters for selecting excipients for nanoparticle lyophilisation, and at the same time having adequate flowability properties which make possible their further manipulation. The results obtained for these two excipients are presented in the examples below. In the examples, the formulations comprising mannitol and dextran are according to the present invention, while the other formulations are reference formulations.

**Example 1**

**Preparation and characterisation of PEC before and after the addition of excipients**

Placebo nanoparticles without the protein were prepared first. Several primary nanoparticle dispersions having the same NP-composition were prepared and characterised for their particle size, polydispersity index, average scattering intensity and zeta potential. The excipients, i.e. mannitol and dextran in different concentrations and combinations were then added to the NP-dispersion and analysed for their characteristics (Table 1) using the same measurement parameters.

The initial particle size of PEC nanoparticles was in the range 210-240 nm with low Pdl, having high scattering intensity and negative zeta potential (Table 1). The addition of the sugars at low concentrations (i.e., below 4 w/v %) showed negligible influence on particle size ($D_f/D_i$ index), whereas dextran at a concentration of about 4 w/v % had a noticeable impact on the particle size, which is most likely due to altered medium parameters (viscosity, refractive index) influencing the data analysis. The zetasizer settings, however, were kept constant for all sample measurements.

**Table 1.** Particle size, polydispersity index, average scattering intensity and zeta potential of PECs before and after the addition of mannitol and/or dextran in different concentrations.

| Dispersion of PEC with excipients (sugar conc, w/v %, in primary dispersion) | | Before addition of excipients | | | | After addition of excipients | | | | $D_f/D_i$ index |
|---|---|---|---|---|---|---|---|---|---|---|
| | | Size (nm) | Pdl | Average Scattering (kcps) | Zeta (mV) | Size (nm) | Pdl | Average Scattering (kcps) | Zeta (mV) | |
| 1 % | M | 217 | 0.23 | 155,795 | -18 | 218 | 0.21 | 136,266 | -18 | 1.00 |
| | D | 219 | 0.20 | 153,460 | -17 | 235 | 0.25 | 131,496 | -16 | 1.07 |
| 2 % | M | 218 | 0.22 | 143,797 | -18 | 229 | 0.22 | 125,450 | -18 | 1.05 |
| | D | 244 | 0.33 | 176,472 | -19 | 252 | 0.22 | 117,537 | -16 | 1.03 |
| 4 % | M | 230 | 0.29 | 117,302 | -18 | 251 | 0.23 | 102,632 | -18 | 1.09 |
| | D | 227 | 0.31 | 173,639 | -18 | 291 | 0.21 | 92,650 | -14 | 1.28 |
| 2% | M + D | 211 | 0.22 | 159,330 | -21 | 233 | 0.21 | 146,584 | -17 | 1.10 |
| 3% | M + D | 246 | 0.27 | 197,352 | -19 | 263 | 0.24 | 157,619 | -17 | 1.07 |
| 4% | M + D | 234 | 0.25 | 157,222 | -18 | 364 | 0.29 | 160,947 | -15 | 1.56 |
| D - dextran, M - mannitol; $D_f/D_i$ index (the ratio between the final and initial mean diameter); size (nm) reflects the mean particle diameter. If M+D was used, the ratio M:D was 1:1. | | | | | | | | | | |

**Example 2**

**Flowability of spray-freeze-dried (SFD) product**

Flowability of the SFD powder was determined with two methods: angle of repose and Carr's compressibility index.

Prior to determining the flowability properties, SFD lyophilisates were passed through a sieve with openings of 1 mm. 200 mg of SFD powder was then transferred to a glass funnel with a diameter of 4.8 cm, which was clamped in the stand so that the lowest part of the funnel was 2.5 cm above the work surface. After the powder flew through the funnel it formed a cone. Angle of repose was determined by measuring the height and radius of the cone of the powder using the equation $\alpha = \tan^{-1}(h/r)$.

When determining Carr's compressibility index (CI) weighted amount of SFD powder (200 mg) was loosely filled into a 25 ml graduated cylinder to monitor the bulk volume and then subjected to 1250 taps in a mechanical shaker (Erweka SVM 10, Germany). Tapped volume was monitored thereafter and Carr's compressibility index was calculated using the equation:

$$CI = (1 - \frac{\rho_{bulk}}{\rho_{tap}}) \times 100\% = (\frac{V_{bulk} - V_{tap}}{V_{bulk}}) \times 100\%$$

The generally accepted scale of flowability is presented in Table 2 (PhEur., US Pharmacopoeia).

**Table 2.** Scale of flowability.

| Flowability properties | Angle of repose (°) | Carr's index (%) |
|---|---|---|
| Excellent | 25-30 | ≤10 |
| Good | 31-35 | 11-15 |

(continued)

| Flowability properties | Angle of repose (°) | Carr's index (%) |
|---|---|---|
| Fair | 36-40 | 16-20 |
| Passable | 41-45 | 21-25 |
| Poor | 46-55 | 26-31 |
| Very poor | 56-65 | 32-37 |
| Very very poor | >66 | >38 |

In addition, descriptive evaluation was made on how SFD powders behave on handling. The most difficult for manipulation were lyophilisates that were highly electrostatic and voluminous or hygroscopic with tendency to dissolve on air humidity.

Results showed that lyophilisates (SFD powders) with mannitol provided better flowability characteristics compared to those containing dextran when tested alone (Table 3). Angle of repose as well as Carr's index were noticeably smaller for mannitol compared to dextran for all tested concentrations. However, the main shortage of lyophilisates with mannitol was their high electrostatic potential, which made them difficult to handle. Lyophilisates with dextran alone had poorer flowability and a high bulk volume, they were also very hygroscopic and unsuited for handling, in particular for further formulation into solid dosage forms.

The combination of both sugars significantly improved the characteristics of the lyophilisates (Table 3). The flowability of the lyophilisates containing mannitol and dextran was similar to that of mannitol lyophilisates but were less electrostatic and more suitable for manipulation.

Mannitol and a combination of mannitol and dextrane both at a concentration 4 w/v % (total) in the primary NP-dispersion (4.8 w/w % NPs per dried product) exhibited excellent flowability properties with respect to both methods of evaluation according to generally accepted scale of flowability.

**Table 3.** Angle of repose, Carr's index and descriptive evaluation of behaviour of lyophilisates (SFD powder) containing mannitol and dextran, either alone or in combination (mannitol:dextran 1:1). Also, the percentage of NPs in SFD powder obtained was calculated.

| SFD PEC with mannitol/dextran (sugar conc, w/v %, in primary dispersion) | | % NPs in SFD powder | Angle of repose (°) | Carr index (%) | Behaviour* |
|---|---|---|---|---|---|
| 1 % | M | 16.7 | 12.9 | 9.1 | E5, H1 |
| | D | | 21.4 | 20.0 | E2, H5 |
| 2 % | M | 9.1 | 20.3 | 18.8 | E5, H1 |
| | D | | 31.5 | 31.3 | E3, H5 |
| 4 % | M | 4.8 | 7.9 | 9.1 | E4, H1 |
| | D | | 42.8 | 22.6 | E3, H5 |
| 2% | M + D | 9.1 | 23.7 | 12.5 | E2, H3 |
| 3% | M + D | 6.3 | 17.6 | 12.5 | E2, H2 |
| 4% | M + D | 4.8 | 9.7 | 9.1 | E1, H2 |
| *Descriptive evaluation of powder behaviour: E- electrostatic; E1 (the least) - E5 (the most electrostatic); H - hygroscopic, H1 (the least) - H5 (the most hygroscopic), D - dextran, M - mannitol. | | | | | |

## Example 3

### SEM images of SFD powder

Scanning electron microscopy (SEM) was used for the morphological evaluation of the SFD powder using a JSM-7001F Jeol (Japan) instrument with an acceleration voltage of 1.5 kV and a secondary electron detector. The SEM images were taken of the SFD nanoparticles containing different excipient(s), which were deposited on a double-sided carbon tape (diameter 12mm, Oxford instruments, Oxon, UK) and then analysed.

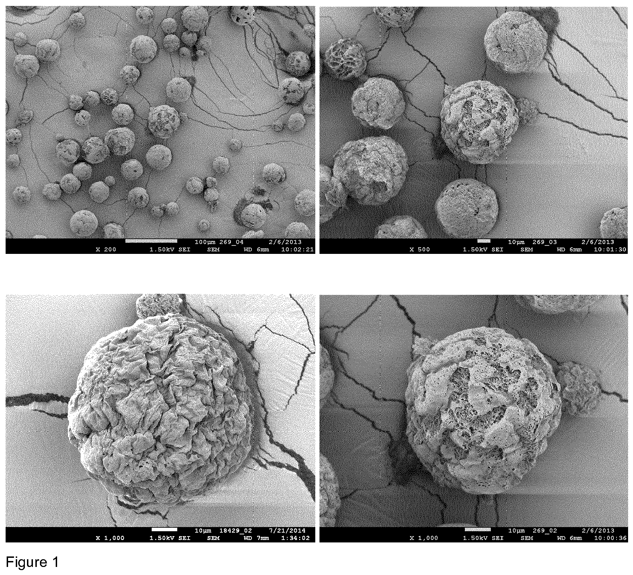

SEM images of SFD particles containing PECs with different excipients are presented in Figures 1-3. The concentration of the sugars (mannitol, dextran and combination of mannitol and dextran) in the nanoparticle dispersion before SFD was 4 w/v%.

SFD particles containing NPs with mannitol alone

SFD particles containing NPs with mannitol alone resulted in spherical particles having porous structure and corrugated surface with closed and open porous parts (Figure 1). The particles had a diameter of about 30-60 micrometers. The lyophilisate had excellent flowability properties, which might be due to the spherically shaped particles that can flow freely between each other.

SFD particles containing NPs with dextran alone

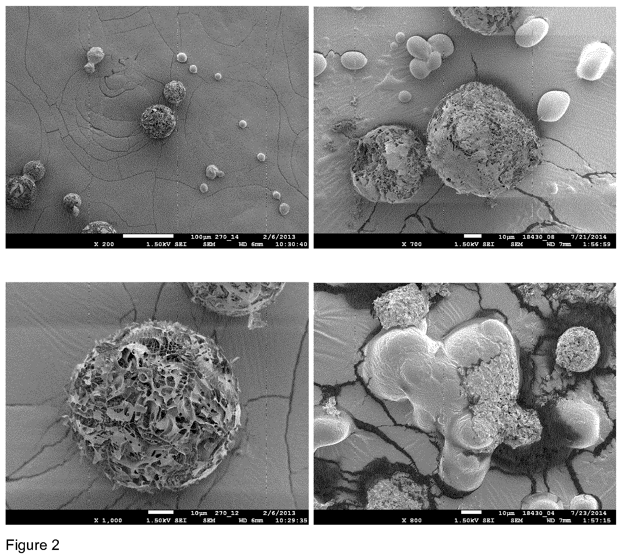

SFD particles containing NPs with dextran alone also resulted in the formation of spherical particles (Figure 2). However, two populations of particles could be observed, the larger particles (approximately 70 $\mu$m) with a highly porous structure and the smaller particles (approximately 20 $\mu$m) with fused and smooth surface. It is believed that due to the high hygroscopic potential of the product containing dextran, particles might bind moisture from the air and could partly dissolve, thus having smaller condensed structure and fused surface area. On the other hand, larger particles showed a highly porous structure with pores that are projected outwards. Their surface is therefore not smooth and could be the reason for poor flowability of the SFD powder, since the particles with projected pores might impede the flow when they move between each other. In addition, such pores seem to have a higher tendency to absorb moisture from air since the SEM image of the sample that was left standing on air for one day (Figure 2, bottom right corner) showed almost completely fused surface area of particles in comparison to SEM image taken on the same but fresh sample one day before. When exposed to air humidity, these particles progressively bind moisture and merged into larger clusters as observed also by reduced bulk volume when standing open on air. Such clustered sample also showed very poor flowability properties.

SFD particles containing NPs with a combination of mannitol and dextran

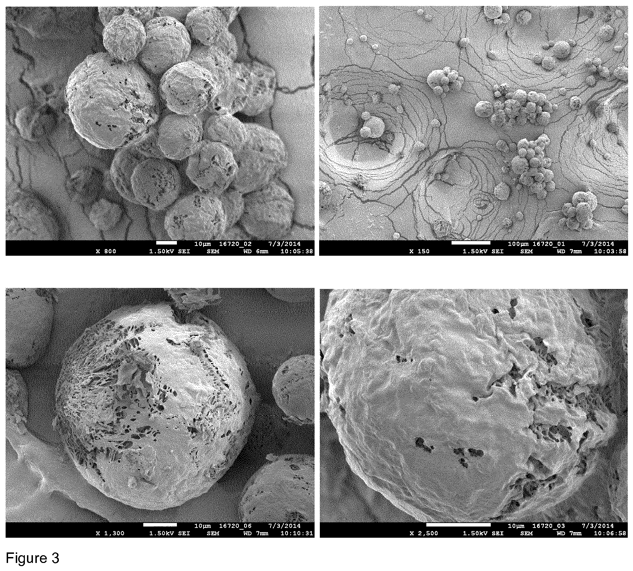

SEM images of SFD nanoparticles containing a combination of mannitol and dextran (1:1) show spherical particles ranging in size from 30 - 60 $\mu$m. The structure of the particles was more similar to that of mannitol alone, i.e., having a porous structure and opened and closed surface area (see Figure 3). Pores that projected outwards particles cannot be observed as in case of SFD with dextran, therefore a better flowability in comparison to that of SFD dextran was observed.

**Example 4**

**Redispersion of SFD powder (lyophilisates)**

Reconstitution of the SFD powder was performed in water during stirring on a magnetic stirrer for 20 min. The volume of water was adjusted to reach a similar concentration of NPs as in the starting dispersion. Visual observation of possible aggregates as well as DLS/ELS measurement were performed on rehydrated lyophilisates.

Lyophilisates that fully redispersed after reconstitution in water are presented in Table 4. Only lyophilisates made from primary dispersions comprising dextran 2 w/v %, or mannitol 4 w/v %, or dextran 4 w/v %, or a combination of mannitol and dextran in concentrations of 3 w/v % and 4 w/v % were able to fully re-disperse in water, thus providing nanoparticle dispersions with the characteristic milky look as indicated by a high average scattering intensity. The particle size of PECs in these dispersions was larger in comparison to the particle size of PECs in dispersions before SFD; however the increase was only by factor of 1.2-1.7. The best redispersion was achieved with a lyophilisate prepared from primary dispersions comprising mannitol and dextran in a concentration of 4 w/v % ($D_f/D_i$ index 1.17 and low PdI 0.21).

Other lyophilisates re-dispersed only partly after reconstitution in water, releasing more or less larger or visible aggregates some of which sedimented with time, thus leaving a dispersion with a more transparent appearance (lower scattering intensity). The desired properties of the nanosystem in these cases were lost.

**Table 4.** Particle size, polydispersity index, average scattering intensity and zeta potential of PEC after reconstitution of SFD powder in water. Particle size and PdI of PEC before SFD are also presented for comparison purposes.

| SFD PECs with M/D (sugar w/v % in primary dispersion) | | Before SFD | | After redispersion of SFD powder in water | | | | | |
|---|---|---|---|---|---|---|---|---|---|
| | | conc, Size (nm) | Pdl | Size (nm) | Pdl | Average scattering (kcps) | Zeta (mV) | $D_f/D_i$ | Full R* |
| 1 % | M | 218 | 0.21 | 832 | 0.38 | 29,551 | -33 | 3.82 | **No** |
| | D | 235 | 0.25 | 556 | 0.26 | 61,142 | -33 | 2.37 | **No** |
| 2 % | M | 229 | 0.22 | 890 | 0.44 | 77,327 | -34 | 3.88 | **No** |
| | D | 252 | 0.22 | 387 | 0.36 | 130,874 | -26 | 1.54 | **Yes** |
| 4 % | M | 251 | 0.23 | 401 | 0.25 | 108,382 | -30 | 1.60 | **Yes** |
| | D | 291 | 0.21 | 407 | 0.40 | 123,895 | -21 | 1.40 | **Yes** |
| 2% | M + D | 233 | 0.21 | 747 | 0.51 | 97,115 | -21 | 3.21 | **No** |
| 3% | M + D | 263 | 0.24 | 453 | 0.36 | 113,105 | -25 | 1.72 | **Yes** |
| 4% | M + D | 364 | 0.29 | 424 | 0.21 | 121,430 | -26 | 1.17 | **Yes** |

M - mannitol, D - dextran; $D_f/D_i$ index (the ratio between the final and initial mean diameter of PEC); Full R* - full redispersion of SFD powder; size (nm) reflects the mean particle diameter. If M+D was used, the ratio M:D was 1:1.

## Example 5

### Redispersion of tablets compressed from the SFD powder

Tablets were compressed from those SFD lyophilisates that fully redispersed after reconstitution in water. An excenter tablet press (Korsch EK-0, Germany) was used for tableting the lyophilisates, which were manually filled into the die mould. Flat punches with 9 mm diameter were used for compressing the powder into tablet. The compression force was 3 kN.

Reconstitution of the tablet compressed from the SFD powder was performed in water during stirring on the magnetic stirrer for 1h. The volume of water was adjusted to reach a similar concentration of NPs as in the starting dispersion. Visual observation of possible aggregates as well as DLS/ELS measurement were performed on rehydrated samples.

Only tablets compressed from lyophilisates made from primary dispersions comprising dextran 2 w/v % or 4 w/v %, or the combination of mannitol and dextran in a total concentration of 4 w/v % in the primary dispersion, were able to fully re-disperse into NPs after reconstitution of tablet in water. The experiment was repeated with additional tablets and showed similar results, full redispersion only for tablets presented in Table 5. Interestingly, in most cases particle size in the reconstituted dispersion was a slightly lower in comparison to starting dispersion.

**Table 5.** Particle size, polydispersity index, average scattering intensity and zeta potential of PEC after reconstitution of tablet compressed from SFD powder in water. Particle size and PdI of PEC before SFD are also presented for comparison purposes.

| Tablet compressed from SFD PEC with D/M (sugar conc, w/v % in primary dispersion): | | Before SFD | | After redispersion of tablet from SFD in water | | | | | |
|---|---|---|---|---|---|---|---|---|---|
| | | Size (nm) | Pdl | Size (nm) | Pdl | Average scattering (kcps) | Zeta (mV) | $D_f/D_i$ | Full R* |
| 2 % | D | 252 | 0.22 | 234 | 0.38 | 156,822 | -22 | 0.93 | **Yes** |
| 4 % | M | 251 | 0.23 | 224 | 0.26 | 98,980 | -30 | 0.89 | **No** |
| | D | 291 | 0.21 | 173 | 0.32 | 137,803 | -22 | 0.59 | **Yes** |
| 3 % | M + D | 263 | 0.24 | 399 | 0.46 | 103,675 | -21 | 1.52 | **No** |

(continued)

| Tablet compressed from SFD PEC with D/M (sugar conc, w/v % in primary dispersion): | | Before SFD | | After redispersion of tablet from SFD in water | | | | | |
|---|---|---|---|---|---|---|---|---|---|
| | | Size (nm) | Pdl | Size (nm) | Pdl | Average scattering (kcps) | Zeta (mV) | $D_f/D_i$ | Full R* |
| 4 % | M+D | 364 | 0.29 | 306 | 0.3 | 111,678 | -30 | 0.84 | **Yes** |
| M - mannitol, D - dextran; $D_f/D_i$ index (the ratio between the final and initial mean diameter of PEC). Full R* - full redispersion of tablet compressed from SFD powder. Size (nm) reflects the mean particle diameter. If M+D was used, the ratio M:D was 1:1. | | | | | | | | | |

## Example 6

### Preparation and characterisation of SFD particles containing protein loaded PEC with excipients

All experiments as presented in examples 1-5 were also performed with G-CSF loaded nanoparticles for the preselected concentrations of mannitol and dextran in the primary PEC nanoparticle dispersion, and the results are presented in Tables 6 and 7.

Results with GCSF-loaded PECs were in line with the previous observations, i.e., good flowability was observed for SDF lyophilisate made from primary dispersions comprising mannitol and a combination of mannitol and dextran (Table 6). The best flowability was achieved with SFD particles prepared from a primary dispersion comprising a combination of mannitol and dextran in a concentration of 4 w/v % (5.1 w/w% NPs per dried product). This powder was also the most suitable for further manipulation. It was the least electrostatic among all tested formulations. Also, the hygroscopic potential of dextran was reduced when combining it with mannitol.

**Table 6.** Flowability and behaviour characteristics of SFD particles containing protein-loaded PEC with mannitol and dextran as excipients.

| SFD PEC with excipients (sugar conc, w/t %, in primary dispersion): | | % NPs in SFD powder | Flowability of SFD powder | | Behaviour |
|---|---|---|---|---|---|
| | | | Angle of repose (°) | Carr index (%) | |
| 2% | D | 9.7 | 28.2 | 29.3 | E3, H4 |
| 4% | D | 5.1 | 50 | 37.8 | E4, H5 |
| 4% | M | 5.1 | 21.8 | 16.7 | E3, H1 |
| 2% | M+D | 9.7 | 20.5 | 5 | E2, H3 |
| 4% | M+D | 5.1 | 4.6 | 5 | E1, H2 |
| M - mannitol; D - dextran; descriptive evaluation or powder behaviour: E - electrostatic; E1 (the least) - E5 (the most electrostatic); H - hygroscopic, H1 (the least) - H5 (the most hygroscopic). If M+D was used, the ratio M:D was 1:1. | | | | | |

Redispersion properties of the SFD powder or of the compressed tablet are presented in Table 7. Redispersion of the SFD powder in water was complete for all presented compositions of the lyophilisates except for the sample prepared from primary dispersions comprising 2 w/v % dextran + mannitol, where large aggregates were observed as sediment.

Redispersion of tablets compressed from SFD powder was the same as evaluated with placebo NPs (Table 5), namely complete redispersion of tablets prepared from SFD powder using 2 w/v% dextran, or 4 w/v % dextran or 4 w/v % dextran + mannitol in the primary dispersion. The particle size of PECs after reconstitution of the tablets in water increased only by a factor 1.1 and 1.3 for tablets containing the SFD powder prepared from a primary dispersion comprising 4 w/v % dextran, or 4 w/v % dextran and mannitol, respectively.

When comparing mannitol and dextran as excipients for SFD of NPs, it is evident that dextran provided better redispersibility of the lyophilisate in comparison to mannitol alone. Mannitol on the other hand is crucial in providing better flowability characteristics of the SFD product.

**Table 7.** Redispersion properties of SFD powder and tablet compressed from SFD powder.

| SFD PEC with M/D (sugar conc., w/t % in primary dispersion) | | Before SFD | | Redispersion of SFD powder | | | | | Redispersion of tablet compressed from SFD powder | | | | |
|---|---|---|---|---|---|---|---|---|---|---|---|---|---|
| | | Size (nm) | Pdl | Size (nm) | Pdl | Average Scattering (kcps) | Df/Di | Full R* | Size (nm) | Pdl | Average Scattering (kcps) | Df/Di | Full R* |
| 2% | D | 303 | 0.39 | 468 | 0.44 | 95,082 | 1.54 | **Yes** | 519 | 0.47 | 83,480 | 1.71 | **Yes** |
| 4% | D | 363 | 0.37 | 451 | 0.45 | 107,318 | 1.24 | **Yes** | 372 | 0.38 | 116,847 | 1.13 | **Yes** |
| 4% | M | 260 | 0.36 | 591 | 0.39 | 74,244 | 2.27 | **Yes** | 507 | 0.36 | 39,970 | 1.95 | **No** |
| 2% | M+D | 263 | 0.35 | 728 | 0.70 | 61,975 | 2.76 | **No** | 547 | 0.43 | 22,704 | 2.07 | **No** |
| 4% | M+D | 328 | 0.38 | 449 | 0.34 | 89,019 | 1.23 | **Yes** | 482 | 0.44 | 108,460 | 1.32 | **Yes** |
| M - mannitol; D - dextran; full R* - full redispersion of SFD powder/tablet; Size (nm) reflects the mean particle diameter. If M+D was used, the ratio M:D was 1:1. | | | | | | | | | | | | | |

**Example 7**

**Biological activity of released protein after rehydration of SDF powder or compressed tablet**

Polyelectrolyte complexes loaded with G-CSF were prepared to evaluate the impact of spray-freeze-drying on protein biological activity. Association efficiency (AE) of G-CSF in PEC was calculated by determining un-associated protein in the supernatant after centrifugation of initial dispersion at 40,000 rpm for 20 minutes (Optima L-100 XP Ultracentrifuge, Beckman Coulter, USA). AE of GCSF in PEC was determined to be 89.8%.

Biological activity of G-CSF released from PEC was determined in reconstituted dispersions after rehydration of the SFD powder- or tablet in water (ca. 2 mg PEC/ml) following 3h release in PBS (1:2, v/v, dispersion/PBS) at 37°C during stirring on a magnetic stirrer. 1 ml samples were taken from the incubating dispersion and centrifuged at 40,000 rpm for 20 minutes. The supernatants were analysed for protein content (RP-HPLC analysis) and the biological activity of G-CSF. The results are presented in Table 8.

**HPLC analysis of protein**

The G-CSF concentration was determined with reversed phase chromatography (RP-HPLC) on a Waters HPLC system. RP-HPLC was carried out on C18, YMCPack ODS-AQ, 4.6 mm $\times$ 15 cm column (YMC, Japan) with mobile phase A (MPA) 10% acetonitrile (ACN), 0.1% trifluoroacetic acid (TFA), mobile phase B (MPB) 90% ACN, 0.1% TFA and a linear gradient from 5% - 100% MPB in 45 min, at 1 mL/min, 35°C. Excitation and emission wavelengths were 280 nm and 345 nm, respectively.

**Biological activity of GCSF**

The in vitro biological activity of released G-CSF was determined by a proliferation assay using NFS-60 cells. The cells were grown in RPMI 1640 medium with 10% FBS (100 % relative humidity, 37°C and 5% $CO_2$). One day prior to testing, the cells were transferred to fresh medium and the next day the cells were washed three times with RPMI 1640 medium with 10% FBS and seeded to 96-well microtiter plates at a density of $1 \times 10^4$ cells per well. Cells were treated with G-CSF standard and samples in serial dilutions. The plates were incubated at 37°C in 5% $CO_2$ incubator for 48 h. The proliferative response was determined by addition of 20 $\mu$l of Presto blue reagent (Molecular Probes). After 3 hour incubation the fluorescence of the resulting product was measured. In vitro biological activity was calculated using a PLA-1.1 program (Stegmann Systems).

Table 8 shows the G-CSF release and biological activity in reconstituted dispersions. The results show that up to 30-40% of the G-CSF was released from PECs in the reconstituted dispersion after 3h release in PBS. Moreover, released G-CSF almost completely retained its biological activity, which indicates that the drying procedure of the present invention as well as further manipulation of the dry product (tableting) did not impair protein biological activity. There is, however, some deviation observed in the biological activity, which is most likely due to variability of the biological assay.

**Table 8.** G-CSF release and biological activity of released protein in reconstituted dispersion prepared from SFD powder or compressed tablet.

| SFD PEC with mannitol and dextran (sugar conc, w/t %, in primary dispersion) | | % NPs in SFD powder | Dosage form | G-CSF release after 3 h in PBS at 37°C (%) | Retained biological activity of G-CSF (%) |
|---|---|---|---|---|---|
| 2% | Dextran | 9.7 | SFD powder | 31.3 | 117.6 |
| | | | Tablet | 35.1 | 106.84 |
| 4% | Dextran | 5.1 | SFD powder | 43.3 | 83.3 |
| | | | Tablet | 27.1 | 83.2 |
| 4% | Mannitol | 5.1 | SFD powder | 42.9 | 82.8 |
| 2% | Dextran + Mannitol (1:1) | 9.7 | SFD powder | 31.8 | 81.2 |

(continued)

| SFD PEC with mannitol and dextran (sugar conc, w/t %, in primary dispersion) | | % NPs in SFD powder | Dosage form | G-CSF release after 3 h in PBS at 37°C (%) | Retained biological activity of G-CSF (%) |
|---|---|---|---|---|---|
| 4% | Dextran + Mannitol (1:1) | 5.1 | SFD powder | 38.6 | 109.5 |
| | | | Tablet | 35.1 | 100.10 |

The SFD process according to the present invention proves to be a suitable procedure for drying PEC nanoparticles containing sensitive protein drugs. With the selection of suitable excipients in accordance with the present invention it is possible to tailor the properties of the final product, i.e., the SFD powder or tablet.

From the experiments presented in examples 1 to 7 it can be concluded that mannitol alone ensures good flowability properties of the SFD product but does not provide adequate re-dispersibility after rehydration of the SFD product in water. If dextran is used as the sole excipient, an SFD product is obtained which shows good re-dispersion in water but with poor flowability and difficult to manipulate. The combination of mannitol and dextran seems to be the best choice for spray-freeze-drying PECs in order to safeguard all requirements as tested, which are good flowability properties, ease of manipulation, and complete re-dispersion in water.

A minimum concentration of mannitol and dextran necessary to obtain an SFD product that meets all these requirements appears to be about 4 w/v % (initial concentration in primary PEC nanoparticle dispersion before SFD), which results in a final product having 5.1 w/w % of NPs per dried powder. In addition, the protein biological activity is preserved during and after the spray-freeze drying procedure according to the present invention, wherein a combination of mannitol and dextran is used as excipient; the biological activity is also retained after tableting.

**Claims**

1.  A process for preparing a spray-freeze-dried powder of polyelectrolyte complex (PEC) nanoparticles comprising the steps of

    (a) providing a primary dispersion comprising PEC nanoparticles,
    at least one sugar that crystallizes during lyophilisation, and
    at least one sugar that remains in the amorphous state,
    wherein said at least one sugar that crystallizes during lyophilisation is mannitol, and said at least one sugar that remains in the amorphous state is dextran,
    (b) spray-freeze drying said primary dispersion, and
    (c) isolating the obtained spray-freeze dried powder,

    wherein said PEC nanoparticles comprise one or more protein drugs.

2.  The process according to claim 1, wherein in said primary dispersion, the total amount of said at least one sugar that crystallizes during lyophilisation and said at least one sugar that remains in the amorphous state is from 2 w/v % to 20 w/v %, or from 3 w/v % to 15 w/v %, or from 3 w/v % to 10 w/v %, or from 3 w/v % to 9 w/v %, or from 3 w/v % to 8 w/v %, or from 3 w/v % to 7 w/v %, or from 3 w/v % to 6 w/v % or from 3 w/v % to 5 w/v %, or from 4 w/v % to 5 w/v %, or 2 w/v %, 3 w/v %, 4 w/v %, 5 w/v %, 6 w/v %, 7 w/v %, 8 w/v %, 9 w/v %, 10 w/v %, 11 w/v %, 12 w/v %, 13 w/v %, 14 w/v %, 15 w/v %, 16 w/v %, 17 w/v %, 18 w/v %, 19 w/v % or 20 w/v %, preferably from 3 w/v % to 5 w/v %, most preferably about 4 w/v %.

3.  The process according to any one of claims 1 or 2, wherein in said primary dispersion, the ratio of said at least one sugar that crystallizes during lyophilisation to said at least one sugar that remains in the amorphous state is from 1:2 to 2:1, or any ratio selected from 1:2, 1.1:1.9, 1.2:1.8, 1.3:1.7, 1.4:1.6, 1:1, 1.6:1.4, 1.7:1.3, 1.8:1.2, 1.9:1 and 2:1, preferably the ratio is 1:1.

4.  The process according to any one of the preceding claims, wherein in said primary dispersion, the total amount of mannitol and dextran is from 2 w/v % to 6 w/v %, preferably from 3 w/v % to 5 w/v %, and most preferably about 4 w/v %.

5. The process according to any one of the preceding claims, wherein in said primary dispersion, the ratio of mannitol to dextran is 1:2 to 2:1, or any ratio selected from 1:2, 1.1:1.9, 1.2:1.8, 1.3:1.7, 1.4:1.6, 1:1, 1.6:1.4, 1.7:1.3, 1.8:1.2, 1.9:1 and 2:1, preferably the ratio is 1:1.

6. The process according to any one of the preceding claims, wherein said at least one sugar that crystallizes during lyophilisation is mannitol, said at least one sugar that remains in the amorphous state is dextran, and wherein in said primary dispersion, the total amount of mannitol and dextran is about 4 w/v%, and the ratio of mannitol and dextran is 1:1.

7. The process of claim 6, wherein said one or more protein drug is selected from G-CSF and EPO, preferably, wherein said protein drug is G-CSF.

8. The process according to any one of the preceding claims, further comprising a step d) of converting said spray-freeze dried powder of step c) into a pharmaceutical composition.

9. A spray-freeze dried powder obtained by the process according to any one of claims 1 to 7.

10. The spray-freeze dried powder according to claim 9, wherein the particles have a mean particle size of from 10 nm to 500 $\mu$m, preferably from 100 nm to 300 $\mu$m, more preferably from 1 $\mu$m to 200 $\mu$m.

11. A pharmaceutical composition comprising the spray-freeze dried powder obtained by the process according to any one of claims 1 to 7.

12. The pharmaceutical composition according to claim 11 obtained by the process according to claim 8.

13. The process according to claim 8, or the pharmaceutical composition according to claims 11 or 12, wherein said pharmaceutical composition is a solid dosage form, or wherein said pharmaceutical composition is a tablet or a capsule.

14. The spray-freeze dried powder according to any one of claims 9 or 10, or the pharmaceutical composition according to any one of claims 11 to 13, wherein the mean particle size determined by light scattering techniques of the particles obtained after redispersion of said spray-freeze dried powder, or of said pharmaceutical composition, in water is less than 1000 nm, preferably less than 500 nm.

15. The spray-freeze dried powder according to any one of claims 9, 10 or 14, or the pharmaceutical composition according to any one of claims 11 to 14, for use in therapy or diagnosis.

16. Use of the spray-freeze dried powder according to any one of claims 9, 10 or 14, or of the pharmaceutical composition according to any one of claims 11 to 14 for the preparation of a medicament.


**Patentansprüche**

1. Verfahren zum Herstellen eines sprühgefriergetrockneten Pulvers von Polyelektrolytkomplex (PEC)-Nanopartikeln, das folgende Schritte umfasst:

   (a) Bereitstellen einer primären Dispersion, die PEC-Nanopartikel umfasst, mindestens eines Zuckers, der während der Lyophilisation kristallisiert, und mindestens eines Zuckers, der im amorphen Zustand verbleibt, wobei der mindestens eine Zucker, der während der Lyophilisation kristallisiert, Mannitol ist, und der mindestens eine Zucker, der im amorphen Zustand verbleibt, Dextran ist,
   (b) Sprühgefriertrocknen der primären Dispersion, und
   (c) Isolieren des erhaltenen sprühgefriergetrockneten Pulvers,

   wobei die PEC-Nanopartikel einen oder mehrere Protein-Arzneimittelstoffe umfassen.

2. Verfahren nach Anspruch 1, wobei in der primären Dispersion der Gesamtanteil des mindestens eines Zuckers, der während der Lyophilisation kristallisiert und des mindestens eines Zuckers, der im amorphen Zustand verbleibt, von 2 m/v% bis 20 m/v%, oder von 3 m/v% bis 15 m/v%, oder von 3 m/v% bis 10 m/v%, oder von 3 m/v% bis 9 m/v%, oder von 3 m/v% bis 8 m/v%, oder von 3 m/v% bis 7 m/v%, oder von 3 m/v% bis 6 m/v% oder von 3 m/v% bis 5 m/v%, oder von 4 m/v% bis 5 m/v%, oder 2 m/v%, 3 m/v%, 4 m/v%, 5 m/v%, 6 m/v%, 7 m/v%, 8 m/v%, 9 m/v%, 10 m/v%, 11 m/v%, 12 m/v%, 13 m/v%, 14 m/v%, 15 m/v%, 16 m/v%, 17 m/v%, 18 m/v%, 19 m/v% oder 20 m/v%, vorzugsweise von 3 m/v% bis 5 m/v%, am meisten bevorzugt 4 m/v% beträgt.

3. Verfahren nach einem der Ansprüche 1 oder 2, wobei in der primären Dispersion das Verhältnis von dem mindestens einen Zucker, der während der Lyophilisation kristallisiert, zu dem mindestens einen Zucker, der im amorphen Zustand verbleibt, von 1:2 bis 2:1 beträgt, oder jedes beliebige Verhältnis ausgewählt aus 1:2, 1.1:1,9, 1,2:1,8, 1,3:1,7, 1,4:1,6, 1:1, 1,6:1,4, 1,7:1,3, 1,8:1,2, 1,9:1 und 2:1, vorzugsweise beträgt das Verhältnis 1:1.

4. Verfahren nach einem der Ansprüche, wobei in der primären Dispersion der Gesamtanteil von Mannitol und Dextran von 2 m/v% bis 6 m/v%, vorzugsweise von 3 m/v% bis 5 m/v% und am meisten bevorzugt etwa 4 m/v% beträgt.

5. Verfahren nach einem der vorstehenden Ansprüche, wobei in der primären Dispersion das Verhältnis von Mannitol zu Dextran 1:2 bis 2:1 beträgt, oder jedes beliebige Verhältnis ausgewählt aus 1:2, 1.1:1,9, 1,2:1,8, 1,3:1,7, 1,4:1,6, 1:1, 1,6:1,4, 1,7:1,3, 1,8:1,2, 1,9:1 und 2:1, vorzugsweise beträgt das Verhältnis 1:1.

6. Verfahren nach einem der vorstehenden Ansprüche, wobei der mindestens eine Zucker, der während der Lyophilisation kristallisiert, Mannitol ist, der mindestens eine Zucker, der im amorphen Zustand verbleibt, Dextran ist, und wobei in der primären Dispersion der Gesamtanteil an Mannitol und Dextran etwa 4 M/v% beträgt und das Verhältnis von Mannitol und Dextran 1:1 beträgt.

7. Verfahren nach Anspruch 6, wobei der eine oder die mehreren Protein-Arzneimittelstoff(e) ausgewählt ist bzw. sind aus G-CSF und EPO, wobei vorzugsweise der Protein-Arzneimittelstoff G-CSF ist.

8. Verfahren nach einem der vorstehenden Ansprüche, das ferner einen Schritt d) des Umwandelns des sprühgefriergetrocknetes Pulvers aus Schritt c) in eine pharmazeutische Zusammensetzung umfasst.

9. Sprühgefriergetrocknetes Pulver, das durch das Verfahren nach einem der Ansprüche 1 bis 7 erhalten wurde.

10. Sprühgefriergetrocknetes Pulver nach Anspruch 9, wobei die Partikel eine mittlere Partikelgröße von 10 nm bis 500 $\mu$m, vorzugsweise von 100 nm bis 300 $\mu$m, mehr bevorzugt von 1 $\mu$m bis 200 $\mu$m aufweisen.

11. Pharmazeutische Zusammensetzung, die das sprühgefriergetrocknete Pulver umfasst, das durch das Verfahren nach einem der Ansprüche 1 bis 7 erhalten wurde.

12. Pharmazeutische Zusammensetzung nach Anspruch 11 die durch das Verfahren nach Anspruch 8 erhalten wurde.

13. Verfahren nach Anspruch 8, oder die pharmazeutische Zusammensetzung nach den Ansprüchen 11 oder 12, wobei die pharmazeutische Zusammensetzung eine feste Dosierungsform ist oder wobei die pharmazeutische Zusammensetzung eine Tablette oder eine Kapsel ist.

14. Sprühgefriergetrocknetes Pulver nach einem der Ansprüche 9 oder 10, oder die pharmazeutische Zusammensetzung nach einem der Ansprüche 11 bis 13, wobei die mittlere Partikelgröße gemäß Messung durch Lichtstreuverfahren der Partikel, die nach Redispersion des sprühgefriergetrockneten Pulvers erhalten wird, oder der pharmazeutischen Zusammensetzung in Wasser weniger als 1000 nm, vorzugsweise weniger als 500 nm beträgt.

15. Sprühgefriergetrocknetes Pulver nach einem der Ansprüche 9, 10 oder 14, oder die pharmazeutische Zusammensetzung nach einem der Ansprüche 11 bis 14, zur Verwendung in Therapie oder Diagnose.

16. Verwendung des sprühgefriergetrockneten Pulvers nach einem der Ansprüche 9, 10 oder 14, oder der pharmazeutischen Zusammensetzung nach einem der Ansprüche 11 bis 14 zur Herstellung eines Medikaments.

**Revendications**

1. Procédé pour préparer une poudre pulvérisée-lyophilisée de nanoparticules de complexe polyélectrolytique (CPE) comprenant les étapes de

EP 3 193 838 B1

(a) fourniture d'une dispersion primaire comprenant des nanoparticules de CPE,
au moins un sucre qui cristallise pendant la lyophilisation et
au moins un sucre qui reste dans l'état amorphe,
ledit au moins un sucre qui cristallise pendant la lyophilisation étant le mannitol et ledit au moins sucre qui reste dans l'état amorphe étant le dextran,
(b) pulvérisation-lyophilisation de ladite dispersion primaire et
(c) isolement de la poudre pulvérisée-lyophilisée obtenue,

lesdites nanoparticules de CPE comprenant un ou plusieurs médicaments protéiniques.

2. Procédé selon la revendication 1, la quantité totale dudit au moins un sucre qui cristallise pendant la lyophilisation et dudit au moins un sucre qui reste dans l'état amorphe, dans ladite dispersion primaire, étant de 2% P/V à 20% P/V ou de 3% P/V à 15% P/V ou de 3% P/V à 10% P/V ou de 3% P/V à 9% P/V ou de 3% P/V à 8% P/V ou de 3% P/V à 7% P/V ou de 3% P/V à 6% P/V ou de 3% P/V à 5% P/V ou de 4% P/V à 5% P/V ou de 2% P/V, de 3% P/V, de 4% P/V, de 5% P/V, de 6% P/V, de 7% P/V, de 8% P/V, de 9% P/V, de 10% P/V, de 11% P/V, de 12% P/V, de 13% P/V, de 14% P/V, de 15% P/V, de 16% P/V, de 17% P/V, de 18% P/V, de 19% P/V ou de 20% P/V, de préférence de 3% P/V à 5% P/V, le plus préférablement d'environ 4% P/V.

3. Procédé selon l'une quelconque des revendications 1 ou 2, le rapport dudit au moins un sucre qui cristallise pendant la lyophilisation audit au moins un sucre qui reste dans l'état amorphe, dans ladite dispersion primaire, étant de 1:2 à 2:1 ou un quelconque rapport choisi parmi 1:2, 1,1:1,9, 1,2:1,8, 1,3:1,7, 1,4:1,6, 1:1, 1,6:1,4, 1,7:1,3, 1,8:1,2, 1,9:1 et 2:1, le rapport étant de préférence 1:1.

4. Procédé selon l'une quelconque des revendications précédentes, la quantité totale de mannitol et de dextran, dans ladite dispersion primaire, étant de 2% P/V à 6% P/V, de préférence de 3% P/V à 5% P/V et le plus préférablement d'environ 4% P/V.

5. Procédé selon l'une quelconque des revendications précédentes, le rapport de mannitol au dextran, dans ladite dispersion primaire, étant de 1:2 à 2:1 ou un quelconque rapport choisi parmi 1:2, 1,1:1,9, 1,2:1,8, 1,3:1,7, 1,4:1,6, 1:1, 1,6:1,4, 1,7:1,3, 1,8:1,2, 1,9:1 et 2:1, le rapport étant de préférence 1:1.

6. Procédé selon l'une quelconque des revendications précédentes, ledit au moins un sucre qui cristallise pendant la lyophilisation étant le mannitol, ledit au moins sucre qui reste dans l'état amorphe étant le dextran et, dans ladite dispersion primaire, la quantité totale de mannitol et de dextran étant d'environ 4% P/V et le rapport de mannitol à dextran étant de 1:1.

7. Procédé selon la revendication 6, ledit un ou lesdits plusieurs médicaments protéiniques étant choisis parmi le G-CSF et l'EPO, ledit médicament protéinique étant de préférence le G-CSF.

8. Procédé selon l'une quelconque des revendications précédentes, comprenant en outre une étape d) de conversion de ladite poudre pulvérisée-lyophilisée de l'étape c) en une composition pharmaceutique.

9. Poudre pulvérisée-lyophilisée obtenue par le procédé selon l'une quelconque des revendications 1 à 7.

10. Poudre pulvérisée-lyophilisée selon la revendication 9, les particules présentant une grosseur moyenne de particule de 10 nm à 500 $\mu$m, de préférence de 100 nm à 300 $\mu$m, plus préférablement de 1 $\mu$m à 200 $\mu$m.

11. Composition pharmaceutique comprenant la poudre pulvérisée-lyophilisée obtenue par le procédé selon l'une quelconque des revendications 1 à 7.

12. Composition pharmaceutique selon la revendication 11 obtenue par le procédé selon la revendication 8.

13. Procédé selon la revendication 8 ou composition pharmaceutique selon les revendications 11 ou 12, ladite composition pharmaceutique étant une forme galénique solide ou ladite composition pharmaceutique étant un comprimé ou une capsule.

14. Poudre pulvérisée-lyophilisée selon l'une quelconque des revendications 9 ou 10 ou composition pharmaceutique selon l'une quelconque des revendications 11 à 13, la grosseur moyenne de particule, déterminée par des techniques de diffusion de la lumière, des particules obtenues après redispersion de ladite poudre pulvérisée-lyophilisée ou de ladite composition pharmaceutique dans l'eau étant inférieure à 1000 nm, de préférence inférieure à 500 nm.

15. Poudre pulvérisée-lyophilisée selon l'une quelconque des revendications 9, 10 ou 14 ou composition pharmaceutique selon l'une quelconque des revendications 11 à 14 pour une utilisation en thérapie ou en diagnostic.

16. Utilisation de la poudre pulvérisée-lyophilisée selon l'une quelconque des revendications 9, 10 ou 14 ou composition pharmaceutique selon l'une quelconque des revendications 11 à 14 pour la préparation d'un médicament.

Figure 1

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6284282 B
- US 20090011008 A1